# EUROPÄISCHE PATENTSCHRIFT

(11) **EP 1 182 928 B1**
(45) Veröffentlichungstag und Bekanntmachung des Hinweises auf die Patenterteilung: **20.11.2002**
(21) Anmeldenummer: 00912660.8
(22) Anmeldetag: 30.03.2000
(51) Int. Cl.: A01N 37/44, A01N 37/12, C08J 7/04

(54) **VERFAHREN ZUR HERSTELLUNG MIKROBIZIDER OBERFLÄCHEN DURCH IMMOBILISIERUNG INHÄRENT MIKROBIZID WIRKSAMER MAKROMOLEKÜLE**
METHOD FOR PRODUCING MICROBICIDAL SURFACES BY IMMOBILIZING INHERENTLY MICROBICIDALLY ACTIVE MACROMOLECULES
PROCEDE DE PREPARATION DE SURFACES MICROBICIDES PAR IMMOBILISATION DE MACROMOLECULES A ACTION MICROBICIDE INHERENTE

(30) Priorität: 12.05.1999 DE 19921894
(43) Veröffentlichungstag der Anmeldung: 06.03.2002
(73) Patentinhaber: Creavis Gesellschaft für Technologie und Innovation mbH, 45772 Marl (DE)
(72) Erfinder: OTTERSBACH, Peter, D-51570 Windeck (DE); OLES, Markus, D-45525 Hattingen (DE)
(86) Internationale Anmeldenummer: EP0002798
(87) Internationale Veröffentlichungsnummer: WO00069264

(56) Entgegenhaltungen:
- EP-A- 0 891 708
- EP-A- 0 893 164
- GB-A- 973 294
- US-A- 5 100 689

## Beschreibung

Die Erfindung betrifft ein Verfahren zur Modifizierung der Oberfläche von Polymersubstraten durch Immobilisierung von inhärent mikrobizid wirksamen Makromolekülen, insbesondere zum Zweck der mikrobiziden Ausstattung von Oberflächen.

Die Modifizierung von Kunststoffoberflächen, speziell von technisch genutzen Produkten, ist von großem wirtschaftlichen Interesse, da hierdurch vollkommen neue Verwendungsmöglichkeiten für bereits im Markt etablierte Standardkunststoffe gefunden werden können. Durch die dadurch bedingten Veränderungen der Oberfläche kommt man auf eine effiziente und ökonomische Art zu Produkten mit auf den speziellen Einsatzzweck hin optimierten Grenzflächeneigenschaften. Diese veränderten Eigenschaften können unter anderem zu hydrophilierten, schmutzabweisenden, bedruckbaren, lösemittelbeständigeren und flammhemmeride Oberflächen führen.
Einen Überblick über die vielfaltigen Möglichkeiten zur Eigenschaftsveränderung synthetischer Polymere durch photoinduzierte Pfropfungen liefert Jr. J. C. Arthur in Dev. Polymer Photochem. 2 ( 1981) 39.

Daneben besteht häufig die Notwendigkeit, Oberflächen dahingehend zu aktivieren, daß sie im Anschluß chemische Gruppen enthalten, die als Reaktionszentren für weitere Kopplungsreaktionen zur Verfügung stehen. Beispielsweise ist die Ozonisierung einer Polymeroberfläche zur Bildung oxidierter Reaktionszentren, wie sie z. B. in US 4311573, US 4589964 oder EP 0378511 beschrieben werden, ein derartiges Verfahren.
Ein weiteres Verfahren, wie z.B. in EP 0574352 beschrieben, beruht auf einer Plasmavorbehandlung von Oberflächen und Zugabe von Sauerstoff zur Generierung von Hydroperoxidgruppen, die ihrerseits als Reaktionszentren für nachgeschaltete Prozesse fungieren können. Darüber hinaus ist in US 4731156 ein Plasmavorbehandlungsverfahren beschrieben, das die unmittelbare Aminofünktionalisierung von Oberflächen gestattet.

Alle genannten Verfahren besitzen den Nachteil, daß die Modifizierung der Oberfläche erst durch nachgeschaltete Prozeßschritte ermöglicht wird. Aus ökonomischen Übelegungen heraus wäre eine direkte, aber nichtsdestotrotz dauerhafte Oberflächenmodifizierung in nur einem Prozeßschritt wünschenswert.

Die antimikrobielle Ausstattung von Oberflächen aller Art gewinnt, vor allem unter Berücksichtigung der in dieser Hinsicht wachsenden Verbraucheroachfrage, rapide an Bedeutung. Eine Möglichkeit zur Herstellung entsprechender Oberflächen bietet sich auf Grundlage von inhärent mikrobiziden Polymeren, die auf Polymeroberflächen gepfropft werden, und somit über einen längeren Zeitraum eine nachhaltige Wirksamkeit versprechen.
Entsprechende Polymersysteme werden z.B. in den europäischen Patentanmeldungen 0 862 858 und 0 862 859 näher beschrieben. Einem weiteren Einsatz entsprechender Systeme steht bis heute vor allem die Problematik einer technisch einfachen und nichtsdestotrotz dauerhaften Anbindung an zu modifizierende Oberflächen im Wege.

Eine andere Vorgehensweise gegen oberflächige Bakterienausbreitungen stellt die Einarbeitung antimikrobiell wirkender Substanzen in eine Matrix dar.

Tert.-Butylaminoethylmethacrylat ist ein handelsübliches Monomer der Methacrylatchemie und wird insbesondere als hydrophiler Bestandteil in Copolymerisationen eingesetzt. So wird in EP-PS 0 290 676 der Einsatz verschiedener Polyacrylate und Polymethacrylate als Matrix fiir die Immobilisierung von bakteriziden quaternären Ammoniumverbindungen beschrieben.

Die US-PS 3 592 805 offenbart die Herstellung systemischer Fungizide, wobei perhalogenierte Acetonderivate mit Methacrylatestern, wie z.B. tert.-Butylaminoethylmethacrylat, umgesetzt werden.

In US-PS 4 515 910 wird der Einsatz von Polymeren aus Fluorwasserstoffsalzen von Aminomethacrylaten in der Dentalmedizin beschrieben. Der in den Polymeren gebundene Fluorwasserstoff tritt langsam aus der Polymermatrix aus und soll Wirkungen gegen Karies besitzen.
Aus einem anderen technischen Bereich offenbart US-PS 4 532 269 ein Terpolymer aus Butylmethacrylat, Tributylzinnmethacrylat und tert.-Butylaminoethylmethacrylat. Dieses Polymer wird als antimikrobieller Schiffsanstrich verwendet, wobei das hydrophile tert.-Butylaminoethylmethacrylat die langsame Erosion des Polymers fördert und so das hochtoxische Tributylzinnmethacrylat als antimikrobiellen Wirkstoff freisetzt.
In diesen Anwendungen ist das mit Aminomethacrylaten hergestellte Copolymere nur Matrix oder Trägersubstanz für zugesetzte mikrobizide Wirkstoffe, die aus dem Trägerstoff diffundieren oder migrieren können. Polymere dieser Art verlieren mehr oder weniger schnell ihre Wirkung, wenn an der Oberfläche die notwendige "minimale inhibitorische Konzentration'' (MIK) nicht mehr erreicht wird.
Der vorliegenden Erfindung liegt daher die Aufgabe zugrunde, Polymeroberflächen durch kovalente Anbindung mikrobizider oder antimikrobieller Polymere in nur einem Prozeßschritt zu modifizieren. Es wurde gefunden, daß sich eine derartige Modifizierung durch Immobilisierung von antimikrobiellen Polymeren auf Polymersubstraten in einfacher Weise durchführen läßt.
EP 0 893 164 beschreibt ein Verfahren bei dem ein bakterienabweisendes Polymer mit einem Vernetzer mittels UV-Strahlung oder thermischer Behandlung auf ein Polymersubstrat gepfropft wird.
GB 973 294 offenbart die Herstellung von Copolymeren Latices, die auf Linoleum aufgebracht werden können. Eine kovalente Immobilisierung des Copolymeren auf der Substratoberfläche ist nicht beschrieben.

### Kurzbeschreibung der Erfindung

Gegenstand der vorliegenden Erfindung ist daher ein Verfahren zur antimikrobiellen Beschichtung von Polymersubstraten, wobei ein antimikrobielles Polymer auf einem Polymersubstrat durch Plasmabehandlung immobilisiert wird.
Das erfindungsgemäße Verfahren kann ausgeübt werden, indem zunächst das antimikrobielle bzw. mikrobizide Polymer auf einem Polymersubstrat physisorbiert und anschließend immobilisiert wird. Die Begriffe "antimikrobiell" und "mikrobizid" sind hier als Synonym zu verstehen.
Zur Aufbringung eignen sich die bekannten Verfahren, wie z.B. Tauchen, Spritzen, Rakeln oder Spin-Coating. Das erfindungemäße Verfahren macht es möglich, bekannte und bewährte Materialien und Herstellverfahren weiterzuverwenden. Dies ist insbesondere dann interessant, wenn die mechanischen Eigenschaften der Werkstoffe bzw. der antimikrobiell auszurüstenden Oberflächen von hoher Bedeutung sind.

### Vorteile der Erfindung

Das erfindungsgemäße Verfahren weist eine bemerkenswerte Kombination von Vorteilen auf. So erzielt man mit inhärent mikrobiziden Polymeren auf chemisch sehr verschiedenartigen Substraten dichte und gleichmäßige Beschichtungen von hervorragender Beständigkeit gegenüber Umgebungseinflüssen einschließlich von Lösemitteln und Abriebkräften sowie hoher antimikrobieller Wirksamkeit. Die zu modifizierenden Kunststoffoberflächen müssen keine bestimmte Topographie aufweisen; Objekte mit einer dreidimensionalen Struktur eignen sich ebensogut wie glatte Flächen. Dies ist insbesondere bei der nachträglichen Modifizierung von vorgefertigten Gegenständen von Vorteil. Durch das zur Immobilisierung des antimikrobiellen Polymers eingesetzte Plasmaverfahren, ist die Zahl gleichzeitig zu modifizierender Gegenstände nur durch die Größe der verwendeten Plasmakammer festgelegt, da das Plasma in der Reaktionskammer homogen auf alle Oberflächen einwirkt.

### Polymere Substrate

Zu den polymeren Substraten, deren Oberflächen erfindungsgemäß modifiziert werden, zählen Homo- und Copolymere, beispielsweise Polyolefine, wie Polyethylen (HDPE und LDPE), Polypropylen, Polyisobutylen, Polybutadien, Polyisopren, natürliche Kautschuke und Polyethylen-co-propylen; halogenhaltige Polymere, wie Polyvinylchlorid, Polyvinylidenchlorid, Polychloropren und Polytetrafluorethylen; Polymere und Copolymere aus vinylaromatischen Monomeren, wie Polystyrol, Polyvinyltoluol, Polystyrol-co-vinyltoluol, Polystyrol-coacrylnitril, Polystyrol-co-butadien-co-acrylnitril; Polykondensate, beispielsweise Polyester, wie Potyethylenterephtalat und Polybutylenterephtalat; Polyamide, wie Polycaprolactam, Polylaurinlactam und das Polykondensat aus Hexamethylendiamin und Adipinsäure; Polyetherblockamide, z.B. aus Laurinlactam und Polyethylenglykol mit durchschnittlich 8, 12, oder 16 Ethylenoxygruppem; weiterhin Polyurethane, Polyether, Polycarbonate, Polysulfone, Polyetherketone, Polyesteramide und imide, Polyacrylnitrtil, Polyacrylate und -methacrylate, Silikone. Auch Blends aus zwei oder mehr Polymeren oder Copolymeren lassen sich nach dem erfindungsgemäßen Verfahren an der Oberfläche modifizieren, ebenso wie Kombinationen aus verschiedenen Polymeren, die durch Verkleben, Verschweißen oder Zusammenschelzen miteinander verbunden sind, einschließlich der Übergangsstellen.

### Inhärent mikrobizide Polymere

Für das Verfahren eignen sich die verschiedenartigsten Polymere mit inhärent mikrobiziden Eigenschaften. Dazu gehören neben Polymerverbindungen mit Stickstoffgruppen, insbesondere quartären Stickstoffgruppen, vor allem die in den europäischen Patentanmeldungen 0 862 858 und 0 862 859 beanspruchten Polymere und Copolymere von tert.-Butylaminoethylmethacrylat.

Das antimikrobielle Polymer kann Stickstoffgruppen, insbesondere quartäre Stickstoffgruppen, oder Poly-tert.-Butylaminoethylmethacrylat enthalten. Es ist weiterhin möglich, Copolymere aus Stickstoffgruppen, insbesondere quaternäre Stickstoffgruppen enthaltenden Monomeren und/oder tert.-Butylaminoethylmethacrylat und mindestens einem weiteren Vinylmonomeren im erfindungsgemäßen Verfahren als antimikrobielles Polymer einzusetzen. Als Vinylmonomere können Acrylate oder Methacrylate, z. B. Acrylsäure, tert.-Butylmethacrylat oder Methylmethacrylat, Styrol, Vinylchlorid, Vinylether, Acrylamide, Acrylnitrile, Olefine (Ethylen, Propylen, Butylen, Isobutylen), Allylverbindungen, Vinylketone, Vinylessigsäure, Vinylacetat oder Vinylester eingesetzt werden. Die Stickstoffgruppen der antimikrobiellen Polymere bzw. zu deren Herstellung eingesetzte Monomere können primäre, sekundäre, tertiäre oder quartäre Amine bzw. Stickstoffgruppen sein.

Das im erfindungsgemäßen Verfahren eingesetzte antimikrobielle, stickstoffgruppenhaltige Polymer kann aus mindestens einfach aliphatisch ungesättigten Monomeren der allgemeinen Formel

R¹NRₐ²R_{b}³R_{c}⁴X_{d}

mit
- R¹:: H, verzweigter, unverzweigter oder cyclischer, gesättigter oder ungesättigter Kohlenwasserstoffrest mit bis zu 50 C-Atomen, die durch O-, N- oder S-Atome substituiert sein können,
- R², R³, R⁴:: H, verzweigter, unverzweigter, oder cyclischer, gesättigter oder ungesättigter Kohlenwasserstoffrest mit bis zu 25 C-Atomen, die durch O-, N- oder S-Atome substituiert sein können, wobei R², R³, R⁴ gleich oder verschieden sind,
- X:: F, Cl⁻, Br⁻, I⁻, SO₄²⁻, SO₃²⁻, CH₃SO₃⁻, CH₃CO₂⁻, C₂O₄²⁻, CO₃²⁻, PO₄³⁻, PO₃²⁻, NO₃⁻, NO₂⁻, NO⁻, CN⁻, SCN⁻, CNO⁻, ClO⁻, ClO₂⁻, ClO₃⁻, ClO₄⁻ und
- a, b, c, d:: jeweils 0 oder 1
aufgebaut werden.

Im Folgenden werden einige Monomere als Beispiele genannt, wobei durch entsprechende Wahl der Indices a, b, c und d primäre, sekundäre, tertiäre oder quartäre aminogruppenhaltige Monomere aufgebaut werden können.

Beispiele für primäre Stickstoffgruppen enthaltende Monomere sind 1-Amino-2-propen, N-6-Aminohexyl-2-propenamid, N-3-Aminopropylmethacrylamid-hydrochlorid, Methacrylsäure-2-aminoethylester-hydrochlorid und 3-Aminopropyl-vinylether.

Als Monomerbausteine mit sekundären Stickstoffgruppen eignen sich, neben den in den europäischen Anmeldungen 0 862 858 und 0 862 859 beschriebenen sekundäraminofunktionalisierten Acryl- bzw. Methacrylsäureestern, alle aliphatisch ungesättigten Monomere, die zumindest eine sekundäre Aminofunktion besitzen, wie z.B. 3-Phenylmethylamino-2-butensäureethylester, 3-Ethylamino-2-butensäureethylester, 3-Methylamino-2-butensäureethylester, 3-Methylamino-1-phenyl-2-propen-1-on, 2-Methyl-N-4-methylamino-1-anthrachinoyl-acrylamid, N-9,10-Dihydro-4-(4-methylphenylamino)-9,10-dioxo-1-anthrachinyl-2-methyl-propenamid, 2-Hydroxy-3-(3-triethoxysilylpropylamino)-2-propensäurepropylester, 1-(1-Methylethylamino)-3-(2-(2-propenyl)-phenoxy)-2-propanolhydrochlorid, 3-Phenylamino-3-methyl-2-butensäure-ethylester, 1-(1-Methylethylamino)-3-(2-(2-propenyloxy)-phenoxy)-2-propanolhydrochlorid, 2-Acrylamido-2-methoxyessigsäuremethylester, 2-Acetamidoacrylsäuremethylester, Acrylsäure-tert.-butylamid, 2-Hydroxy-N-2-propenyl-benzamid, N-Methyl-2-propenamid.

Als tertiäre Stickstoffgruppen enthaltende Monomere können z.B. Methacrylsäure-2-diethylaminoethylester, Methacrylsäure-2-dimethylaminoethylester, Methacrylsäure-3-dimethylaminopropylamid, Acrylsäure-2-diethylaminoethylester, Acrylsäure-2-dimethylaminoethylester, Acrylsäure-3-dimethylaminopropylester, Acrylsäure-3-dimemylamino-2,2-dimethylpropylester eingesetzt werden.

Beispiele für quarternäre Stickstoffgruppen enthaltende Monomere sind z.B. 2-Methacryloyloxyethyl-trimethylammoniumchlorid, 2-Methacryloyloxyethyl-trimethylammoniumethosulfat, 3-Methacryloylammopropyl-trimethylammoniumchlorid, N,N,N-trimethyl-3-(2-methyl-1-oxo-2-propenylamino)-1-propanammoniumchlorid; N,N,N-triethyl-2-(1-oxo-2-propenylamino)- ethanammoniumchlorid, N,N,N-trimethyl-ethenammoniumbromid.

Die aus diesen Monomeren hergestellten erfindungsgemäß eingesetzten Polymeren sollten einerseits eine ausreichend hohe Molmasse mit einer ausreichend hohen Anzahl von Aminofunktionen enthalten, andererseits bezüglich Löslichkeit und/oder Schmelzpunkt noch gut handhabbar sein. Polymere mit mehr als 35 Monomereinheiten oder einem Molgewicht über 3000 g/mol sind in der Regel fiir das erfindungsgemäße Verfahren geeignet.

### Vorbehandlung der Substratoberflächen

Die Aufbringung bzw. Physisorption des antimikrobiellen Polymers auf das Polymersubstrat kann durch Auftragen einer Lösung des Polymers erfolgen. Diese Lösung besteht im allgemeinen aus 0,1 bis 80 Gew.-%, vorteilhaft 0,5 bis 50 Gew.-%, des inhärent mikrobiziden bzw. antimikrobiellen Polymers in einem geigneten Lösemittel. Die Wahl des Lösemittels richtet sich u.a. nach der Löslichkeit der Komponenten ineinander und der chemischen Natur des Substrates. Dabei ist es durchaus von Vorteil, wenn die Lösung das Polymersubstrat moderat anquellen kann, um eine noch intensivere Verbindung aus Polymersubstrat und inhärent mikrobizidem Polymer zu erhalten. Auch die Zugabe weiterer Komponenten, wie z. B. Monomere oder Radikalstarter, kann im Einzelfall zu einer weiter verbesserten Anbindung führen.

Die optimalen Kombinationen von Substratpolymer, Lösemittelsystem und inhärent mikrobizidem Polymer lassen sich durch orientierende Versuche unschwer ermitteln. Beispielsweise ist Ethanol in Verbindung mit Poly-tert.-Butylaminoethylmethacrylat gut fiir die Beschichtung von Polyurethansubstraten geeignet.

Falls es sich bei dem aufzubringenden inhärent mikrobiziden Polymer um einen unzersetzt schmelzbaren Thermoplasten handelt, kann die Aufbringung bzw. Physisorption auf dem Polymersubstrat auch durch Auftragung einer Schmelze dieses Polymers erfolgen.

Die Behandlung des Polymersubstrats mit dem im Lösemittelsystem gelösten inhärent mikrobiziden Polymer kann so erfolgen, daß die Oberfläche des Polymersubstrats leicht quillt. Die Behandlungsdauer hängt von der jeweiligen Kombination von Polymersubstrat, inhärent mikrobizidem Polymer und Lösemittelsystem sowie von der Temperatur ab. Sie braucht nur 1 bis 60 Sekunden zu betragen und beträgt vorteilhaft 1 bis 30 Sekunden. Die optimalen Temperaturen und Behandlungszeiten lassen sich unschwer durch orientierende Versuche ermitteln; typische Vorgehensweisen sind in den Beispielen aufgezeigt. Vorzugsweise erfolgt die Behandlung des Polymersubstrats mit dem im Lösemittelsystem gelösten inhärent mikrobiziden Polymer bei einer Temperatur von -20° bis 200°C, besonders bevorzugt bei Temperaturen von 0° bis 80°C und insbesondere bei 10° bis 60°C.

Lösungen aus oder mit dem inhärent mikrobiziden Polymer können durch übliche Beschichtungsverfahren, wie Aufsprühen, Bestreichen oder Tauchen, auf das Polymersubstrat aufgebracht werden.

In vielen Fallen ist es zweckmäßig, vor der Immobilisierung an dem vorbehandelten Substrat anhaftendes Lösemittel zu entfernen. Dies kann z.B. durch kurzzeitiges Trocknen (zweckmäßig einige Sekunden bis zu etwa zehn Minuten) in einem geeigneten Trockenschrank geschehen.

### Immobilisierung

Die auf die Physisorbtion des inhärent mikrobiziden Polymers folgende Immobilisierung erfolgt durch Plasmabehandlung (RF- oder Mikrowellenplasma). Die Expositionszeiten betragen im allgemeinen 3 bis 300 Sekunden. Die Expositionszeiten hängen u.a. von Zusammensetzung der Beschichtungsmischung ab. Man arbeitet im Allgemeinen mit RF-Plasmen (RF = radio frequency) mit ca. 600 bis 1200 Watt Leistung bzw. Mikrowellenplasmen mit ca. 50 Watt Leistung.

### Optionale Nachbehandlung

Nach der Plasmabehandlung kann man etwaige unerwünschte Lösemittelreste oder Restmonomere durch Extraktion mit einem Lösemittel, wie z.B. Wasser, entfernen. Weiterhin lassen sich eingeführte Gruppen in üblicher Weise ganz oder teilweise in Derivate überführen. So kann man Carboxylgruppen zu Carboxylatgruppen neutralisieren, Carbonestergruppen zu Hydroxyl- oder Carbonsäuregruppen sowie Carbonamid- oder Nitrilgruppen zu Carboxylgruppen verseifen. Weitere Derivatisierungen von erfindungsgemäß modifizierten Polymersubstraten können nach allgemeinen Verfahren (H. Beyer, Lehrbuch der organischen Chemie, S. Hirzel Verlag, Stuttgart, 1988, S. 260 ff) vorgenommen werden.

### Verwendung der modifizierten Polymersubstrate

Weitere Gegenstände der vorliegenden Erfindung sind die Verwendung von erfindungsgemäß modifizierten Polymersubstraten zur Herstellung von antimikrobiell wirksamen Erzeugnissen, insbesondere medizintechnische Artikel, und die so hergestellten Erzeugnisse als solche. Die Erzeugnisse können erfindungsgemäß modifizierte Polymersubstrate enthalten oder aus diesen bestehen. Solche Erzeugnisse basieren vorzugsweise auf Polyamiden, Polyurethanen, Polyetherblockamiden, Polyesteramiden oder -imiden, PVC, Polyolefinen, Silikonen, Polysiloxanen, Polymethacrylat oder Polyterephthalaten, die erfindungsgemäß modifizierte Oberflächen aufweisen, vorzugsweise mit quartären Stickstoffgruppen oder, wie im Fall des Poty-tert.-Butylaminoethylmethacrylates und seiner Copolymere, als mikrobizid wirksam bekannte Amingruppen.

Antimikrobiell wirksame Erzeugnisse dieser Art sind beispielsweise und insbesondere Maschinenteile für die Lebensmittelverarbeitung, Bauteile von Klimaanlagen, Bedachungen, Badezimmer- und Toilettenartikel, Küchenartikel, Komponenten von Sanitäreinrichtungen, Komponenten von Tierkäfigen - und behausungen, Spielwaren, Komponenten in Wassersystemen, Lebensmittelverpackungen, Bedienelemente (Touch Panel) von Geräten oder Kontaktlinsen.

Die durch das erfindungsgemäße Verfahren hergestellten Beschichtungen können überall verwendet werden, wo es auf möglichst bakterienfreie d.h. mikrobizide Oberflächen oder Oberflächen mit Antihafteigenschaften ankommt. Als Verwendungsbeispiele für nach dem erfindungsgemäßen Verfahren mikrobizid beschichtete Polymersubstrate bzw. Oberflächen seien insbesondere Beschichtungen in den folgenden Bereichen genannt:
- Marine: Schiffsrümpfe, Hafenanlagen, Bojen, Bohrplattformen, Ballastwassertanks
- Haus: Bedachungen, Keller, Wände, Fassaden, Gewächshäuser, Sonnenschutz, Gartenzäune, Holzschutz
- Sanitär: Öffentliche Toiletten, Badezimmer, Duschvorhänge, Toilettenartikel, Schwimmbad, Sauna, Fugen, Dichtmassen
- Lebensmittel: Maschinen, Küche, Küchenartikel, Schwämme, Spielwaren, Lebensmittelverpackungen, Milchverarbeitung, Trinkwassersysteme, Kosmetik
- Maschinenteile: Klimaanlagen, Ionentauscher, Brauchwasser, Solaranlagen, Wärmetauscher, Bioreaktoren, Membranen
- Medizintechnik: Kontaktlinsen, Windeln, Membranen, Implantate
- Gebrauchsgegenstände: Autositze, Kleidung (Strümpfe, Sportbekleidung), Krankenhauseinrichtungen, Türgriffe, Telefonhörer, öffentliche Verkehrsmittel, Tierkäfige, Registrierkassen, Teppichboden, Tapeten

Außerdem sind Gegenstände der vorliegenden Erfindung die Verwendung der erfindungsgemäß an der Oberfläche modifizierten Polymersubstrate zur Herstellung von Hygieneerzeugnissen und die Hygieneerzeugnisse als solche. Die obigen Ausführungen über bevorzugte Materialien gelten entsprechend. Solche Hygieneerzeugnisse sind beispielsweise Zahnbürsten, Toilettensitze, Kämme und Verpackungsmaterialien. Unter die Bezeichnung Hygieneartikel fallen auch andere Gegenstände, die mit vielen Menschen in Berührung kommen, wie Telefonhörer, Handläufe von Treppen, Tür- und Fenstergriffe sowie Haltegurte und -griffe in öffentlichen Verkehrsmitteln.

Zur weiteren Beschreibung der vorliegenden Erfindung werden die folgenden Beispiele gegeben, die die Erfindung weiter erläutern, nicht aber ihren Umfang begrenzen sollen, wie er in den Patentansprüchen dargelegt ist.

### Beispiel 1:

90 ml tert-Butylaminoethylmethacrylat (Fa. Aldrich) und 180 ml Ethanol werden in einen Dreihalskolben vorgelegt und unter Argonzustrom auf 65° C erhitzt. Danach werden 0,74 g Azobisisobutyronitril gelöst in 20 ml Ethylmethylketon unter Rühren langsam zugetropft. Das Gemisch wird auf 70° C erhitzt und bei 48 Stunden bei dieser Temperatur gerührt. Nach Ablauf dieser Zeit wird die Reaktionsmischung in 1 Liter Wasser eingerührt, wobei das polymere Produkt ausfällt. Nach Abfiltrieren des Produktes wird der Filterrückstand mit 250 ml Cyclohexan gespült, um noch vorhandene Restmonomere zu entfernen. Im Anschluß wird das Produkt fiir 24 Stunden bei 50° C im Vakuum getrocknet.

### Beispiel 2:

2 g Poly-tert.-Butylaminoethylmethacrylat aus Beispiel 1 werden in 8 ml Ethanol gelöst. Diese Lösung wird in ein verschließbares Reagenzglas gefüllt.

Ein Polyurethanschlauch (3 mm Durchmesser, 5 cm Länge) wird an beiden Enden mit einer Gasflamme zugeschmolzen. Die Auftragung des Polymers auf den Schlauch erfolgt durch Eintauchen des Schlauches in das mit der Lösung gefüllte Reagenzglas bei 22° C für 20 Sekunden Der vorbehandelte Schlauch wird mit seinen Enden so auf Befestigungsblöcke aus Stahl gelegt, daß der Schlauch nur am Anfangs- und Endpunkt aufliegt. Der Schlauch wird für 2 Minuten im Trockenschrank bei 50° C getrocknet und anschließend in die Plasmakammer gegeben. Die Plasmakammer wird verschlossen und es wird ein Vakuum angelgt. Danach wird ein Argonstrom von 0,3 l/min eingestellt, die Plasmaleistung auf 600 Watt einreguliert und der Schlauch für die Dauer von 30 Sekunden plasmabehandelt.

Nach Beendigung der Behandlung wird der Schlauch entnommen und für zwei Stunden bei 40° C getrocknet.

### Beispiel 3:

Ein Schlauch aus Beispiel 2 wird in 20 ml einer Testkeimsuspension von Staphylococcus aureus eingelegt und geschüttelt. Nach einer Kontaktzeit von 15 Minuten wird 1 ml der Testkeimsuspension entnommen, und die Keimzahl im Versuchsansatz bestimmt. Nach Ablauf dieser Zeit sind keine Keime von Staphylococcus aureus mehr nachweisbar.

### Beispiel 4:

Ein Schlauch aus Beispiel 2 wird in 20 ml einer Testkeimsuspension von Pseudomonas aeruginosa eingelegt und geschüttelt. Nach einer Kontaktzeit von 60 Minuten wird 1 ml der Testkeimsuspension entnommen, und die Keimzahl im Versuchsansatz bestimmt. Nach Ablauf dieser Zeit ist die Keimzahl von 10⁷ auf 10⁴ abgefallen.

### Beispiel 5:

90 ml 2-Methacryloyloxyethyl-trimethylammoniumchlorid (Fa. Aldrich) und 180 ml entmineralisiertes Wasser werden in einen Dreihalskolben vorgelegt und unter Argonzustrom auf 65° C erhitzt. Danach werden 0,4 g Natriumperoxodisulfat gelöst in 15 ml entmineralisiertem Wasser unter Rühren langsam zugetropft. Das Gemisch wird auf 70° C erhitzt und bei 48 Stunden bei dieser Temperatur gerührt. Nach Ablauf dieser Zeit wird die Reaktionsmischung in 1 1 n-Hexan eingerührt, wobei das polymere Produkt ausfällt. Nach Abfiltrieren des Produktes wird der Filterrückstand mit 50 ml einer äquimolaren Ethanol/Wasser-Lösung gespült, um noch vorhandene Restmonomere zu entfernen. Im Anschluß wird das Produkt für 24 Stunden bei 50° C im Vakuum getrocknet.

### Beispiel 6:

2 g Poly-2-Methacryloyloxyethyl-trimethylammoniumchlorid aus Beispiel 5 werden in 15 ml entmineralisiertem Wasser gelöst. Diese Lösung wird in ein verschließbares Reagenzglas gefüllt.

Ein Polyurethanschlauch (3 mm Durchmesser, 5 cm Länge) wird an beiden Enden mit einer Gasflamme zugeschmolzen. Die Auftragung des Polymers auf den Schlauch erfolgt durch Eintauchen des Schlauches in das mit der Lösung gefüllte Reagenzglas bei 22° C für 20 Sekunden Der vorbehandelte Schlauch wird mit seinen Enden so auf Befestigungsblöcke aus Stahl gelegt, daß der Schlauch nur am Anfangs- und Endpunkt aufliegt. Der Schlauch wird für 2 Minuten im Trockenschrank bei 70° C getrocknet und anschließend in die Plasmakammer gegeben. Die Plasmakammer wird verschlossen und es wird ein Vakuum angelegt. Danach wird ein Argonstrom von 0,3 l/min eingestellt, die Plasmaleistung auf 600 Watt einreguliert und der Schlauch fiir die Dauer von 30 Sekunden plasmabehandelt.

Nach Beendigung der Behandlung wird der Schlauch entnommen und fiir zwei Stunden bei 40° C getrocknet.

### Beispiel 7:

Ein Schlauch aus Beispiel 6 wird in 20 ml einer Testkeimsuspension von Staphylococcus aureus eingelegt und geschüttelt. Nach einer Kontaktzeit von 15 Minuten wird 1 ml der Testkeimsuspension entnommen, und die Keimzahl im Versuchsansatz bestimmt. Nach Ablauf dieser Zeit sind keine Keime von Staphylococcus aureus mehr nachweisbar.

### Beispiel 8:

Ein Schlauch aus Beispiel 6 wird in 20 ml einer Testkeimsuspension von Pseudomonas aeruginosa eingelegt und geschüttelt. Nach einer Kontaktzeit von 60 Minuten wird 1 ml der Testkeimsuspension entnommen, und die Keimzahl im Versuchsansatz bestimmt. Nach Ablauf dieser Zeit ist die Keimzahl von 10⁷ auf 10³ abgefallen.

Zusätzlich zur oben beschriebenen mikrobiziden Wirksamkeit gegenüber Zellen von Pseudomonas aeruginosa und Staphylococcus aureus zeigten alle Proben ebenfalls eine mikrobizide Wirkung gegenüber Zellen von Klebsiella pneumoniae, Escherichia coli, Rhizopus oryzae, Candida tropicalis und Tetrahymena pyriformis.

## Patentansprüche

1. Verfahren zur antimikrobiellen Beschichtung von Polymersubstraten,
**dadurch gekennzeichnet,**
**daß** ein antimikrobielles Polymer auf einem Polymersubstrat durch Plasmabehandlung immobilisiert wird.

2. Verfahren nach Anspruch 1,
**dadurch gekennzeichnet,**
**daß** das antimikrobielle Polymer zunächst auf einem Polymersubstrat physisorbiert und anschließend immobilisiert wird.

3. Verfahren nach Anspruch 2,
**dadurch gekennzeichnet,**
**daß** die Physisorption durch Auftragen einer Lösung oder einer Schmelze des antimikrobiellen Polymers auf das Polymersubstrat erfolgt.

4. Verfahren nach einem der Ansprüche 1 bis 3,
**dadurch gekennzeichnet,**
**daß** das antimikrobielle Polymer Stickstoffgruppen enthält.

5. Verfahren nach einem der Ansprüche 1 bis 4,
**dadurch gekennzeichnet,**
**daß** das antimikrobielle Polymer quartäre Stickstoffgruppen enthält.

6. Verfahren nach einem der Ansprüche 1 bis 5,
**dadurch gekennzeichnet,**
**daß** das antimikrobielle Polymer Poly-tert.butylaminoethylmethacrylat enthält.

7. Verfahren nach einem der Ansprüche 1 bis 6,
**dadurch gekennzeichnet,**
**daß** das antimikrobielle Polymer ein Copolymer eines Stickstoffgruppen enthaltenden Monomeren und mindestens eines weiteren Vinylmonomeren ist.

8. Verfahren nach einem der Ansprüche 1 bis 7,
**dadurch gekennzeichnet,**
**daß** das antimikrobielle Polymer ein Copolymer eines quartäre Stickstoffgruppen enthaltenden Monomers und mindestens eines weiteren Vinylimonomeren ist.

9. Verfahren nach einem der Ansprüche 1 bis 8,
**dadurch gekennzeichnet,**
**daß** das antimikrobielle Polymer ein Copolymer von tert.-Butylaminoethylmethacrylat und mindestens einem weiteren Vinylmonomeren ist.

10. Verwendung der gemäß den Ansprüchen 1 bis 9 hergestellten antimikrobiellen Beschichtungen zur Herstellung von Hygieneartikeln.

11. Verwendung der gemäß den Ansprüchen 1 bis 9 hergestellten antimikrobiellen Beschichtungen zur Herstellung von medizintechnischen Artikeln.

## Claims

1. A process for the antimicrobial coating of a polymer substrate, **characterized in that** it comprises immobilizing an antimicrobial polymer on a polymer substrate by plasma treatment.

2. A process according to claim 1, **characterized in that** the antimicrobial polymer is firstly physisorbed on a polymer substrate and then immobilized.

3. A process according to claim 2, **characterized in that** the physisorption takes place by applying a solution or a melt of the antimicrobial polymer to the polymer substrate.

4. A process according to any one of claim 1 to 3, **characterized in that** the antimicrobial polymer contains nitrogen groups.

5. A process according to any one of claims 1 to 4, **characterized in that** the antimicrobial polymer contains quaternary nitrogen groups.

6. A process according to any one of claims 1 to 5, **characterized in that** the antimicrobial polymer comprises poly-tert-butylaminoethyl methacrylate.

7. A process according to any one of claims 1 to 6, **characterized in that** the antimicrobial polymer is a copolymer of a monomer containing a nitrogen group and at least one other vinyl monomer.

8. A process according to any one of claims 1 to 7, **characterized in that** the antimicrobial polymer is a copolymer of a monomer containing a quaternary nitrogen group and at least one other vinyl monomer.

9. A process according to any one of claims 1 to 8, **characterized in that** the antimicrobial polymer is a copolymer of tert-butylaminoethyl methacrylate and at least one other vinyl monomer.

10. The use of the antimicrobial coatings produced according to any of claims 1 to 9 for producing hygiene items.

11. The use of the antimicrobial coatings produced according to any of claims 1 to 9 for producing items for medical technology.

## Revendications

1. Procédé de revêtement antimicrobien de substrats polymères,
**caractérisé en ce qu'**
un polymère antimicrobien est immobilisé sur un substrat polymère par traitement par plasma.

2. Procédé selon la revendication 1,
**caractérisé en ce que**
le polymère antimicrobien est absorbé physiquement en premier lieu sur un substrat polymère et ensuite est immobilisé,

3. Procédé selon la revendication 2,
**caractérisé en ce que**
l'absorption physique s'effectue par application d'une solution ou d'un produit de fusion du polymère antimicrobien sur le substrat polymère.

4. Procédé selon l'une quelconque des revendications 1 à 3,
**caractérisé en ce que**
le polymère antimicrobien contient des groupes azotés.

5. Procédé selon l'une quelconque des revendications 1 à 4,
**caractérisé en ce que**
le polymère antimicrobien renferme des groupes azotés quaternaires.

6. Procédé selon l'une quelconque des revendications 1 à 5,
**caractérisé en ce que**
le polymère antimicrobien renferme du polyméthacrylatc de terbutylaminoéthyle.

7. Procédé selon l'une quelconque des revendications 1 à 6,
**caractérisé en ce que**
le polymère antimicrobien est un copolymére de monomères contenant un groupe azoté et d'au moins un autre monomère vinylique.

8. Procédé selon l'une quelconque des revendications 1 à 7,
**caractérisé en ce que**
le polymère antimicrobien est un copolymère d'un monomère contenant des groupes azotés quaternaires et d'au moins un autre monomère vinylique.

9. Procédé selon l'une quelconque des revendications 1 à 8,
**caractérisé en ce que**
le polymère antimicrobien est un copolymère de méthacrylate de terbutylaminoéthyle et d'au moins un autre monomère vinylique.

10. Utilisation des revêtements antimicrobiens produits conformément aux revendications 1 à 9 pour la production d'articles d'hygiène.

11. Utilisation des revêtements antimicrobiens produits conformément aux revendications 1 à 9 pour la production d'articles de la technique médicale.
